# EUROPEAN PATENT APPLICATION

(11) **EP 3 693 015 A1**
(43) Date of publication of application: **12.08.2020**
(21) Application number: 19219328.2
(22) Date of filing: 26.09.2014
(51) Int. Cl.: A61K 39/395, A61K 39/40, A61K 39/00, A61K 39/38, A61K 39/02, A61K 39/108

(54) **BINDING MOIETIES FOR BIOFILM REMEDIATION**

(30) Priority: 13.01.2014 US 201461926828 P
(62) Divisional of application: 14847993.4
(71) Applicant: Trellis Bioscience, LLC, Redwood City, CA 94063 (US)
(72) Inventor: Kauvar, Lawrence M., San Francisco, CA 94109 (US); Ryser, Stefan, Menlo Park, CA 94025 (US); Estelles, Angeles, Belmont, CA 94002 (US); Simon, Reyna J., Los Gatos, CA 95033 (US); Bakaletz, Lauren Opremcak, Columbus, OH 43205 (US); Goodman, Steven David, Columbus, OH 43205 (US)
(74) Representative: V.O.

(57) **Abstract**

Binding agents able to disrupt bacterial biofilms of diverse origin are described, including monoclonal antibodies secreted by human B lymphocytes. Methods to prevent formation of or to dissolve biofilms with these binding agents are also described. Immunogens for eliciting antibodies to disrupt biofilms are also described.

## Description

### Technical Field

The invention relates to methods and compositions for dissolution of biofilms that inhibit immune responses and make bacteria resistant to antibiotics. More specifically, it concerns monoclonal antibodies that are derived from human cells or from transgenic animals expressing human antibody genes or that are humanized forms of antibodies native to other species wherein the affinity for the proteins that are responsible for the structural integrity of such biofilms exceeds the affinity of these proteins for biofilm components. Monoclonal antibodies in general and other binding moieties with this property are also included.

### Background Art

It is well understood in the art that bacterial infections may lead to formation of biofilms that protect the bacteria from the immune system and lead them to enter a quiescent, slow growth state that makes them resistant to most antibiotics (Donlan, R. M., et al., Clin Microbiol Rev (2002) 15:167-193). The result is persistent, recurrent infections that are very difficult to eliminate. These biofilms include as a major component branched DNA molecules that are held together by specific proteins generally designated DNABII proteins, with homologs found in most bacterial species (Goodman, S. D., et al., Mucosal Immunity (2011) 4:625-637). The substantial homology of these proteins facilitates the cooperative formation of biofilms, a feature that further renders the bacteria problematic from a treatment perspective. The present invention is based on the concept that supplying a binding moiety with sufficiently high affinity for this class of proteins will extract the proteins from the biofilm and thereby provide an effective method of destroying the biofilm by destroying the ability of the protein to bind and hold together the branched DNA. A supplied binding moiety against the DNABII protein may also destroy its ability to bind to other components present in the biofilm.

The binding moieties, of which monoclonal antibodies or fragments thereof are an important embodiment, can be supplied directly to biofilms or used to coat surfaces to provide an immuno-adsorbent for confining the DNABII protein(s). Applications include treatments of bacterial infections by systemic administration, subcutaneous, topical or inhaled administration, as well as reduction of biofouling that affects pipelines and other industrial equipment. Application to corresponding biofilm associated diseases of animals is also part of the present invention.

PCT publication WO2011/123396 provides an extensive discussion of such biofilms and suggests their removal by administering to a subject polypeptides that represent the DNABII protein itself, thus causing the organism to generate antibodies that can destroy the integrity of the biofilm. This document also suggests, in the alternative, supplying the antibodies themselves, either *ex vivo* to biofilms that exist outside an organism or to a subject to confer passive protection.

This PCT application describes the use of polyclonal antibodies generated against a particular DNABII protein (*E. coli* integration host factor (IHF)) to treat an animal model of the common ear infection (otitis media) and of an animal model for periodontal disease. It also describes generating active immunity by providing the protein, or peptides representing the protein to a subject. There is no disclosure of any monoclonal antibodies with the desired affinity that are directed to this protein. Nor is there any disclosure of binding moieties that show cross-species activity against homologs of the IHF protein. Achieving both properties represents a significant obstacle to discovery of an effective drug. The present invention overcomes these obstacles and provides improved agents for passive immunity.

### Disclosure of the Invention

The invention provides homogeneous compositions of binding moieties, such as aptamers, protein mimics or monoclonal antibodies or fragments thereof, that are particularly effective in binding the DNABII protein and thus effective in dissolving biofilms. Thus, the invention in one aspect is directed to a binding moiety such as a monoclonal antibody (mAb) that has affinity for at least one DNABII protein that exceeds the affinity of branched DNA, a component of biofilms, for said protein. It is particularly preferred that any antibodies to be used systemically be compatible with mammalian subjects, especially human subjects or feline, canine, porcine, bovine, ovine, caprine or equine subjects when proposed for use in these subjects. Such native mAbs have lower risk of binding to other proteins in the body than mAbs from other sources and thus pose lower toxicity risk. Similarly, immunogenicity of mAbs native to a subject is expected to be lower than for other mAb sources thereby facilitating repeated administration. Specific binding moieties illustrated herein contain at least the CDR regions of the heavy chains, and optionally the light chains of the mAb's TRL295, TRL1012, TRL1068, TRL1070, TRL1087, TRL1215, TRL1216, TRL1218, TRL1230, TRL1232, TRL1242 and TRL1245. However, other types of binding moieties, such as aptamers, modifications of antibodies such as camel type single-chain antibodies and the like are also included within the scope of the invention.

The invention is further directed to a method to treat a biofilm associated with an industrial process by using the binding moieties of the invention either to dissolve biofilms or prevent their formation. In this instance, a full variability of binding moieties is suitable, and the species origin of mAb's is not of concern. The binding moieties may also be applied topically on a subject to dissolve biofilms or to prevent their formation. The binding moieties may also be administered systematically for treatment of biofilms.

In still other aspects, the invention is directed to recombinant materials and methods to prepare binding moieties of the invention that are proteins, and to improved recombinant methods to prepare DNABII proteins.

In other aspects, the invention is directed to novel expression systems for DNABII proteins to be used as immunogens and to methods to use these DNABII proteins to identify an agent that reverses drug resistance in multiple species of bacteria. The latter methods comprise evaluating agents for binding activity to the DNABII proteins produced by multiple microbial species.

The invention also relates to specific isolated peptides that span predicted immunogenic epitope regions of the IHFα chain of the *E. coli* DNABII as well as to methods for generating antibodies to IHF proteins by using these peptides as immunogens.

In still another aspect, the invention is directed to a method to treat human or animal diseases for which biofilm causes drug resistance. Examples include: heart valve endocarditis (for which surgical valve replacement is required in the substantial fraction of cases that cannot be cured by high dose antibiotics due to the resistance associated with biofilm), chronic non-healing wounds (including venous ulcers and diabetic foot ulcers), ear and sinus infections, urinary tract infections, pulmonary infections (including subjects with cystic fibrosis or chronic obstructive pulmonary disease), catheter associated infections (including renal dialysis subjects), subjects with implanted prostheses (including hip and knee replacements), and periodontal disease. This method is effective in mammalian subjects in general, and thus is also applicable to household pets. For example, 85% of dogs over the age of 3 have periodontal disease which is difficult to treat due to biofilm (Kortegaard, H. E., et al., J. Small Anim. Pract. (2008) 49:610-616). Similarly, the invention has utility for treating farm animals. For example, 98% of cultures from dairy cattle with mastitis display bacterial infections associated with biofilm production: Poliana de Castro Melo, et al., Brazilian J. Microbiology (2013) 44:119-124).

### Brief Description of the Drawings

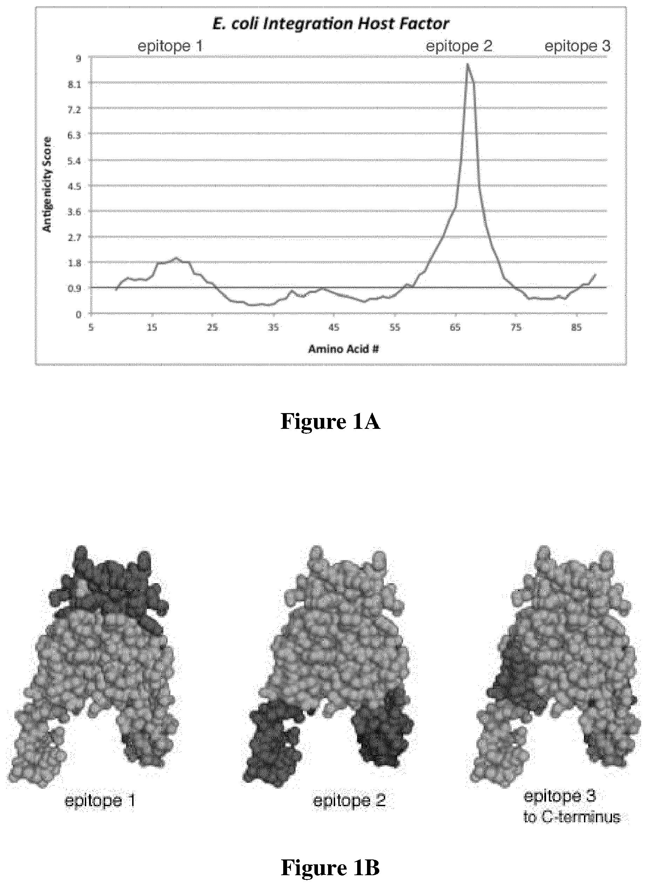

Figure 1A shows the result of a computational analysis of sites on IHF that are likely to be particularly susceptible to antibody attack (scores above 0.9). Residues 10-25, 56-78, and 86-96 of *Haemophilus influenzae* (Hi) IHF are thereby identified as promising targets. Figure 1B shows these likely antigenic sites mapped onto the crystal structure of the IHF protein (based on the Protein Data Bank (pdb) structure designated 1OWF).

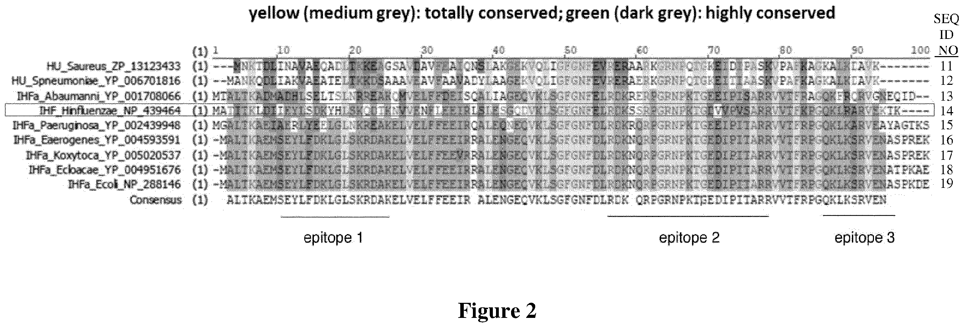

Figure 2 shows the location of the predicted epitopes of the invention in IHF proteins of various bacterial species.

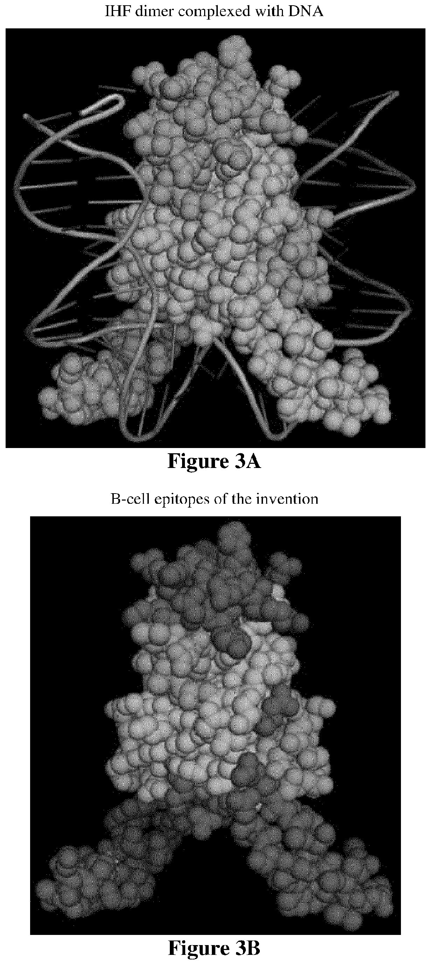

Figure 3A shows a three-dimensional model of IHF proteins in their native dimeric form as complexed with DNA. Figure 3B shows the predicted highly antigenic regions (the darkened regions shown (which are red in the color version). The epitopes 2 and 3 identified in Figure 1 are partially shielded from exposure to the immune system by DNA which is abundant in the biofilm.

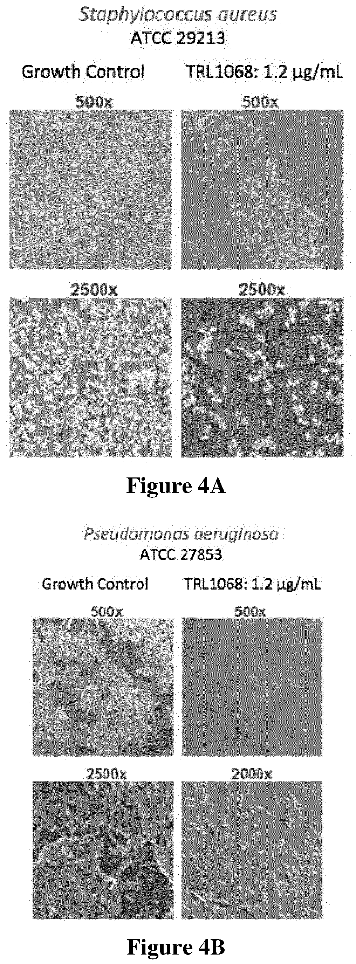

Figure 4A shows *Staphylococcus aureus* (Sa) biofilm treated for 12 hours with an isotype control mAb that does not bind the target protein (growth control) or with TRL1068 at 1.2 µg/mL (-10 nM), a native human mAb against a conserved epitope on DNABII proteins. TRL1068 caused dissolution of the biofilm, as evident at both low (500x) and high (2500x) magnification (scanning electron microscope images). Figure 4B shows the parallel experiment on *Pseudomonas aeruginosa* (Pa) biofilm.

### Modes of Carrying Out the Invention

The invention includes various binding moieties of a monoclonal or homogeneous nature that can dissolve biofilms. "Monoclonal" means that the binding moieties can form a homogeneous population analogous to the distinction between monoclonal and polyclonal antibodies. In one important embodiment, the exemplified binding moieties are mAbs or fragments thereof. In most embodiments, the binding moieties have affinity for at least one DNABII protein in the low nanomolar range - *i*.*e*., the Kd is in the range of 10 nM - 100 nM including the intervening values, such as 25 nM or 50 nM, but may also be < 10 nM as a preferred embodiment.

As the illustrative antibodies disclosed herein in the examples below are derived from humans, the constant regions of these antibodies will be human which offers particular advantages for repeated use in humans. When the subject to be administered the mAb is non-human, it is advantageous for repeated use to administer native mAb's derived from that species. Alternatively, an equivalent of the human variable regions, optionally fused to an Fc region from the host species to be treated, may be used. This variable region may be, in some embodiments, an Fab portion or a single-chain antibody containing CDR regions from both the heavy and light chains. Bispecific forms of these variable regions equivalents can also be constructed, with numerous constructs described in the literature. Although the typical "mAb" will be a protein or polypeptide ("proteins," "polypeptide" and "peptide" are used interchangeably herein without regard to length) for use in subjects, the mAb's may also be supplied via delivery of nucleic acids that then generate the proteins *in situ.* In addition, nucleic acid molecules that mimic the binding characteristics of these polypeptides or proteins can be constructed - *i*.*e*., aptamers can be constructed to bind molecules that are identified as described below by their ability to mimic the binding moieties. Successful mimicry of these aptamers for the protein-based binding moieties can verified both biochemically and functionally to confirm that the affinity of the aptamer is sufficient for therapeutic efficacy.

With respect to protein-based monoclonal binding moieties, in addition to typical monoclonal antibodies or fragments thereof that are immunologically specific for the same antigen, various forms of other scaffolding, including single-chain antibody forms such as those derived from camel, llama or shark could be used as well as antibody mimics based on other scaffolds such as fibronectin, lipocalin, lens crystallin, tetranectin, ankyrin, Protein A (Ig binding domain), or the like. Short structured peptides may also be used if they provide sufficient affinity and specificity, e.g. peptides based on inherently stable structures such as conotoxins or avian pancreatic peptides, or peptidomimetics that achieve stable structures by crosslinking and/or use of non-natural amino acids: Josephson K., et al., JAm Chem Soc (2005) 127(33):11727-11725). In general, "monoclonal antibody (mAb)" includes all of the foregoing.

As used herein, the term "antibody" includes immunoreactive fragments of traditional antibodies even if, on occasion, "fragments" are mentioned redundantly. The antibodies, thus, include Fab, F(ab')₂, Fᵥ fragments, single-chain antibodies which contain a substantially only variable regions, bispecific antibodies and their various fragmented forms that still retain immunospecificity and proteins in general that mimic the activity of "natural" antibodies by comprising amino acid sequences or modified amino acid sequences (*i*.*e*., pseudopeptides) that approximate the activity of variable regions of more traditional naturally occurring antibodies.

For the variable regions of mAb's, as is well known, the critical amino acid sequences are the CDR sequences arranged on a framework which framework can vary without necessarily affecting specificity or decreasing affinity to an unacceptable level. Definition of these CDR regions is accomplished by art-known methods. Specifically, the most commonly used method for identifying the relevant CDR regions is that of Kabat as disclosed in Wu, T. T., et al., J. Exp. Med. (1970) 132:211-250 and in the book Kabat, E. A., et al. (1983) Sequence of Proteins of Immunological Interest, Bethesda National Institute of Health, 323 pages. Another similar and commonly employed method is that of Chothia, published in Chothia, C., et al., J. Mol. Biol. (1987) 196:901-917 and in Chothia, C., et al., Nature (1989) 342:877-883. An additional modification has been suggested by Abhinandan, K. R., et al., Mol. Immunol. (2008) 45:3832-3839. The present invention includes the CDR regions as defined by any of these systems or other recognized systems known in the art.

The specificities of the binding of the mAb's of the invention are defined, as noted, by the CDR regions mostly those of the heavy chain, but complemented by those of the light chain as well (the light chains being somewhat interchangeable). Therefore, the mAb's of the invention may contain the three CDR regions of a heavy chain and optionally the three CDR's of a light chain that matches it. The invention also includes binding agents that bind to the same epitopes as those that actually contain these CDR regions. Thus, for example, also included are aptamers that have the same binding specificity - *i.e*., bind to the same epitopes as do the mAb's that actually contain the CDR regions. Because binding affinity is also determined by the manner in which the CDR's are arranged on a framework, the mAb's of the invention may contain complete variable regions of the heavy chain containing the three relevant CDR's as well as, optionally, the complete light chain variable region comprising the three CDR's associated with the light chain complementing the heavy chain in question. This is true with respect to the mAb's that are immunospecific for a single epitope as well as for bispecific antibodies or binding moieties that are able to bind two separate epitopes, for example, divergent DNABII proteins from two bacterial species.

The mAb's of the invention may be produced recombinantly using known techniques. Thus, with regard to the novel antibodies described herein, the invention also relates to nucleic acid molecules comprising nucleotide sequence encoding them, as well as vectors or expression systems that comprise these nucleotide sequences, cells containing expression systems or vectors for expression of these nucleotide sequences and methods to produce the binding moieties by culturing these cells and recovering the binding moieties produced. Any type of cell typically used in recombinant methods can be employed including prokaryotes, yeast, mammalian cells, insect cells and plant cells. Also included are human cells (*e.g.,* muscle cells or lymphocytes) transformed with a recombinant molecule that encodes the novel antibodies.

Bispecific binding moieties may be formed by covalently linking two different binding moieties with different specificities. For example, the CDR regions of the heavy and optionally light chain derived from one monospecific mAb may be coupled through any suitable linking means to peptides comprising the CDR regions of the heavy chain sequence and optionally light chain of a second mAb. If the linkage is through an amino acid sequence, the bispecific binding moieties can be produced recombinantly and the nucleic acid encoding the entire bispecific entity expressed recombinantly. As was the case for the binding moieties with a single specificity, the invention also includes the possibility of binding moieties that bind to one or both of the same epitopes as the bispecific antibody or binding entity/binding moiety that actually contains the CDR regions.

The invention further includes bispecific constructs which comprise the complete heavy and light chain sequences or the complete heavy chain sequence and at least the CDR's of the light chains or the CDR's of the heavy chains and the complete sequence of the light chains.

The invention is also directed to nucleic acids encoding the bispecific moieties and to recombinant methods for their production.

Multiple technologies now exist for making a single antibody-like molecule that incorporates antigen specificity domains from two separate antibodies (bi-specific antibody). Thus, a single antibody with very broad strain reactivity can be constructed using the Fab domains of individual antibodies with broad reactivity to Group 1 and Group 2 respectively. Suitable technologies have been described by Macrogenics (Rockville, MD), Micromet (Bethesda, MD) and Merrimac (Cambridge, MA). (See, *e.g.,* Orcutt, K. D., Ackerman, M. E., Cieslewicz, M., Quiroz, E., Slusarczyk, A. L., Frangioni, J. V., Wittrup, K. D., "A Modular IgG-scFv Bispecific Antibody Topology," Protein Eng Des Sel. (2010) 23:221-228; Fitzgerald, J., Lugovskoy, A., "Rational Engineering of Antibody Therapeutics Targeting Multiple Oncogene Pathways," MAbs. (2011) 1:3(3); Baeuerle, P. A., Reinhardt, C., "Bispecific T-cell Engaging Antibodies for Cancer Therapy," Cancer Res. (2009) 69:4941-4944.)

The invention also includes a method for identifying suitable immunogens for use to generate antibodies by assessing the binding of the binding moieties of the invention, such as mAb's described above, to a candidate peptide or other molecule. This is an effective method, not only to identify suitable immunogens, but also to identify compounds that can be used as a basis for designing aptamers that mimic the binding moieties of the invention. The method is grounded in the fact that if a vaccine immunogen cannot bind to an optimally effective mAb, it is unlikely to be able to induce such antibodies. Conversely, an immunogen that is a faithful inverse of the optimal mAb provides a useful template for constructing a mimic of the optimal mAb. In its simplest form, this method employs a binding moiety such as one of the mAb's of the invention as an assay component and tests the ability of the binding moiety to bind to a candidate immunogen in a library of said candidates. Successful binders can be used to generate additional antibodies for treating biofilms by administering them to vertebrate subjects such as mice, rabbits, rats, other mammals or avian subjects.

In addition, the ability of the binding moieties of the invention to overcome drug resistance in a variety of bacteria can be assessed by testing the binding moieties of the invention against a panel or library of DNABII proteins from a multiplicity of microbial species. Binding moieties that are able to bind effectively a multiplicity of such proteins are thus identified as suitable not only for dissolving biofilms in general, but also as effective against a variety of microbial strains. It is also useful to identify binding moieties that have utility in acidic environments wherein the affinity of a candidate binding moiety for a DNABII protein over a range of pH conditions is tested and moieties with a low nanomolar affinity at pH 4.5 are identified as having utility in acidic environments.

The binding moieties of the invention are also verified to have an affinity with respect to at least one DNABII protein greater than the affinity of a biofilm component for the DNABII protein which comprises comparing the affinity of the binding moiety for the DNABII protein *versus* the affinity of a component of the biofilm, typically branched DNA, for the DNABII protein. This can be done in a competitive assay, or the affinities can be determined independently.

The DNABII proteins used in these assays may be prepared in mammalian cells at relatively high yield.

All of the assays above involve assessing binding of two perspective binding partners in a variety of formats.

A multitude of assay types are available for assessing successful binding of two prospective binding partners. For example, one of the binding partners can be bound to a solid support and the other labeled with a radioactive substance, fluorescent substance or a colorimetric substance and the binding of the label to the solid support is tested after removing unbound label. The assay can, of course, work either way with the binding moiety attached to the solid support and a candidate immunogen or DNABII protein labeled or *vice versa* where the candidate is bound to solid support and the binding moiety is labeled. Alternatively, a complex could be detected by chromatographic means based on molecular weight such as SDS-page. The detectable label in the context of the binding assay can be added at any point. Thus, if, for example, the mAb or other binding moiety is attached to a solid support the candidate immunogen can be added and tested for binding by supplying a labeled component that is specific for the candidate immunogen. Hundreds of assay formats for detecting binding are known in the art, including, in the case where both components are proteins, the yeast two-hybrid assay.

In addition to this straightforward application of the utility of the binding moieties of the invention, the identification of a suitable powerful immunogen can be determined in a more sophisticated series of experiments wherein a panel of mAbs against the DNABII protein is obtained and ranked in order by efficacy. A full suite of antibodies or other binding moieties can be prepared against all possible epitopes by assessing whether additional binding moieties compete for binding with the previous panel of members. The epitopes for representative binding mAbs for each member of the complete suite can be accomplished by binding to a peptide array representing the possible overlapping epitopes of the immunogen or by X-ray crystallography, NMR or cryo-electron microscopy. An optimal vaccine antigen would retain the spatial and chemical properties of the optimal epitope defined as that recognized by the most efficacious mAbs as compared to less efficacious mAbs but does not necessarily need to be a linear peptide. It may contain non-natural amino acids or other crosslinking motifs.

Moreover, screening can include peptides selected based on their likelihood of being recognized by antibodies and based on their conservation across bacterial species. As described in Example 3 below, for IHF these two criteria have converged on a single peptide - residues 56-78 of *H. influenzae* and corresponding positions in other analogs.

Thus, even beyond the specific mAb's set forth herein, optimal immunogens can be obtained, which not only are useful in active vaccines, but also as targets for selecting aptamers. Specifically, in addition to positions 56-78 of *H. influenzae,* the peptides at positions 10-25 and 86-96 of *H. influenzae* are identified.

Another aspect of the invention is a method to prepare higher yields of the bacterial/microbial DNABII proteins which are typically somewhat toxic to bacteria. The standard method for preparation of these proteins is described by Nash, H. A., et al., J. Bacteriol (1987) 169:4124-4127 who showed that the IHF of *E. coli* could be effectively prepared if both chains of said protein (IHF alpha and IHF beta) are produced in the same transformant. Applicants have found that they are able to obtain higher yields, as much as 5-10 mg/l of IHF, by producing both chains transiently in HEK293 cells. The expression of bacterial proteins that are toxic at high levels in bacteria is conveniently achieved in mammalian cells especially for those without glycosylation sites that would result in modification of the proteins when thus expressed. Purification of the resulting protein can be achieved using fast protein liquid chromatography (FPLC).

### Applications

The binding moieties of the invention including antibodies are useful in therapy and prophylaxis for any subject that is susceptible to infection that results in a biofilm. Thus, various mammals, such as bovine, ovine and other mammalian subjects including horses and household pets and humans will benefit from the prophylactic and therapeutic use of these mAb's.

The binding moieties of the invention may be administered in a variety of ways. The peptides based on CDR regions of antibodies, including bispecific and single chain types or alternate scaffold types, may be administered directly as veterinary or pharmaceutical compositions with typical excipients. Liposomal compositions are particularly useful, as are compositions that comprise micelles or other nanoparticles of various types. Aptamers that behave as binding agents similar to mAb's can be administered in the same manner. Further, the binding agent may be conjugated to any of the solid supports known in the literature, such as PEG, agarose or a dextran, to function as an immuno-sorbent for extracting IHF from a biofilm. Alternatively, the peptide-based mAb's may be administered as the encoding nucleic acids either as naked RNA or DNA or as vector or as expression constructs. The vectors may be non-replicating viral vectors such as adenovirus vectors (AAV) or the nucleic acid sequence may be administered as mRNA packaged in a liposome or other lipid particle. Use of nucleic acids as drugs as opposed to their protein counterparts is helpful in controlling production costs.

These are administered in a variety of protocols, including intravenous, subcutaneous, intramuscular, topical (particularly for chronic non-healing wounds and periodontal disease), inhaled and oral or by suppository. Similar routes of administration can be used with regard to the binding moieties themselves. One useful way to administer the nucleic acid-based forms of either the binding moieties themselves (aptamers) or those encoding the protein form of binding moieties is through a needleless approach such as the agro-jet needle-free injector described in US2001/0171260.

The peptides that represent the epitopes of the IHF proteins as described herein are also useful as active components of vaccines to stimulate immunogenic responses which will generate antibodies *in situ* for disruption of biofilms. The types of administration of these immunogens or peptidyl mimetics that are similarly effective are similar to those for the administration of binding moieties, including various types of antibodies, etc. The peptidomimetics may themselves be in the form of aptamers or alternative structures that mimic the immunogenic peptides described herein. For those immunogens, however, that are proteins or peptides, the administration may be in the form of encoding nucleic acids in such form as will produce these proteins *in situ.* The formulation, routes of administration, and dosages are determined conventionally by the skilled artisan.

The types of conditions for which the administration either of the vaccine type for active generation of antibodies for biofilm control or for passive treatment by administering the antibodies *per se,* include any condition that is characterized by or associated with the formation of biofilms. These conditions include: heart valve endocarditis, both native and implanted (for which a substantial fraction of cases cannot be cured by high dose antibiotics due to the resistance associated with biofilm), chronic non-healing wounds (including venous ulcers and diabetic foot ulcers), ear and sinus infections, urinary tract infections, pulmonary infections (including subjects with cystic fibrosis or chronic obstructive pulmonary disease), catheter associated infections (including renal dialysis subjects), subjects with implanted prostheses (including hip and knee replacements), and periodontal disease.

As noted above, the binding moieties of the invention are not limited in their utility to therapeutic (or diagnostic) uses, but can be employed in any context where a biofilm is a problem, such as pipelines or other industrial settings. The mode of application of these binding moieties to the biofilms in these situations, again, is conventional.

The following examples are offered to illustrate but not to limit the invention.

### Example 1

### Preparation of Antibodies

Human peripheral antibody producing memory B cells were obtained from recovered sepsis patients or from anonymized blood bank donors, under informed consent. The cells were subjected to the CellSpot™ assay to determine their ability to bind the DNABII protein derived from influenza virus. The CellSpot™ assay is described in U.S. patents 7,413,868 and 7,939,344. After isolating the B cells from whole blood, they were stimulated with cytokines and mitogens to initiate a brief period of proliferation and antibody secretion (lasting -10 days) and plated for subjection to the assays; the encoding nucleic acids were extracted and used to produce the antibodies recombinantly.

Antibodies selected based on binding to at least one of the DNABII proteins or fragments thereof were characterized: TRL295, TRL1012, TRL1068, TRL1070, TRL1087, TRL1215, TRL1216, TRL1218, TRL1230, TRL1232, TRL1242 and TRL1245. Affinity was measured using the FortéBio™ Octet™ biosensor to measure on and off rates (whose ratio yields the Kd). This result establishes the feasibility of a focused screen to isolate high affinity, cross-strain binding antibodies.

TRL295 heavy chain variable region has the amino acid sequence:

QVQLVESGGGLVQPGGSLRLSCAASGFPFSSYAMSWVRQAPGKGLEWVSAISGNGADS
YYADSVKGRFTTSRDKSKNTVYLQMNRLRAEDTAVYYCAKDMRRYHYDSSGLHFWG
QGTLVTVSS (SEQ ID NO:1);

TRL295 light chain variable region has the amino acid sequence:

DIELTQAPSVSVYPGQTARITCSGDALPKQYAYWYQQKPGQAPVVVIYKDSERPSGISER
FSGSSSGTTVTLTISGVQAGDEADYYCQSVDTSVSYYVVFGGGTKLTVL (SEQ ID NO:2);

TRL1012 heavy chain variable region has the amino acid sequence:

QVQLVESGGGLVQPGGSLRLSCAASGFPFSSYAMSWVRQAPGKGLEWVSAISGNGADS
YYADSVKGRFTTSRDKSKNTVYLQMNRLRAEDTAVYYCAKDMRRYHYDSSGLHFWG
QGTLVTVSS (SEQ ID NO:3);

TRL1012 light chain variable region has the amino acid sequence:

DIMLTQPPSVSAAPGQKVTISCSGSSSNIGTNYVSWFQQVPGTAPKFLIYDNYKRPSETPD
RFSGSKSGTSATLDITGLQTGDEANYYCATWDSSLSAWVFGGGTKVTVL
(SEQ ID NO:4);

TRL1068 heavy chain variable region has the amino acid sequence:

QVQLVESGPGLVKPSETLSLTCRVSGDSNRPSYWSWIRQAPGKAMEWIGYVYDSGVTIY
NPSLKGRVTISLDTSKTRFSLKLTSVIAADTAVYYCARERFDRTSYKSWWGQGTQVTVS
S (SEQ ID NO:5);

TRL1068 light chain variable region has the amino acid sequence:

DIVLTQAPGTLSLSPGDRATLSCRASQRLGGTSLAWYQHRSGQAPRLILYGTSNRATDTP
DRFSGSGSGTDFVLTISSLEPEDFAVYYCQQYGSPPYTFGQGTTLDIK (SEQ ID NO:6);

TRL1070 heavy chain variable region has the amino acid sequence:

QVQLVQSGGTLVQPGGSLRLSCAASGFTFSYYSMSWVRQAPGKGLEWVANIKHDGTER
NYVDSVKGRFTISRDNSEKSLYLQMNSLRAEDTAVYYCAKYYYGAGTNYPLKYWGQG
TRVTVSS (SEQ ID NO:7);

TRL1070 light chain kappa variable region has the amino acid sequence:

DILMTQSPSSLSASVGDRVTITCRASQGIRNDLGWYQQKPGKAPKLLIYAASSLQSGVPS
RFSGSGSGTDFTLTISSLQPEDFATYYCLQDYNYPLTFGGGTKVEIKR (SEQ ID NO:8);

TRL1087 heavy chain variable region has the amino acid sequence:

QVQLLESGPGLVRPSDTLSLTCTFSADLSTNAYWTWIRQPPGKGLEWIGYMSHSGGRDY
NPSFNRRVTISVDTSKNQVFLRLTSVTSADTAVYFCVREVGSYYDYWGQGILVTVSS
(SEQ ID NO:9);

TRL1087 light chain kappa variable region has the amino acid sequence:

DIEMTQSPSSLSASVGDRITITCRASQGISTWLAWYQQKPGKAPKSLIFSTSSLHSGVPSK
FSGSGSGTDFTLTITNLQPEDFATYYCQQKWETPYSFGQGTKLDMIR (SEQ ID NO:10);

TRL1215 heavy chain variable region has the amino acid sequence:

QVQLVESGTEVKNPGASVKVSCTASGYKFDEYGVSWVRQSPGQGLEWMGWISVYNGK
TNYSQNFQGRLTLTTETSTDTAYMELTSLRPDDTAVYYCATDKNWFDPWGPGTLVTVS
S (SEQ ID NO:11);

TRL1215 light chain lambda variable region has the amino acid sequence:

DIVMTQSPSASGSPGQSITISCTGTNTDYNYVSWYQHHPGKAPKVIIYDVKKRPSGVPSR
FSGSRSGNTATLTVSGLQTEDEADYYCVSYADNNHYVFGSGTKVTVL (SEQ ID NO:12);

TRL1216 heavy chain variable region has the amino acid sequence:

QVQLVESGGGVVQPGGSLRVSCAASAFSFRDYGIHWVRQAPGKGLQWVAVISHDGGK
KFYADSVRGRFTISRDNSENTLYLQMNSLRSDDTAVYYCARLVASCSGSTCTTQPAAFD
IWGPGTLVTVSS (SEQ ID NO:13);

TRL1216 light chain lambda variable region has the amino acid sequence:

DIMLTQPPSVSVSPGQTARITCSGDALPKKYTYWYQQKSGQAPVLLIYEDRKRPSEIPER
FSAFTSWTTATLTITGAQVRDEADYYCYSTDISGDIGVFGGGTKLTVL (SEQ ID NO:14);

TRL1218 heavy chain variable region has the amino acid sequence:

QVQLLESGADMVQPGRSLRLSCAASGFNFRTYAMHWVRQAPGKGLEWVAVMSHDGY
TKYYSDSVRGQFTISRDNSKNTLYLQMNNLRPDDTAIYYCARGLTGLSVGFDYWGQGT
LVTVSS (SEQ ID NO:15);

TRL1218 light chain lambda variable region has the amino acid sequence:

DIVLTQSASVSGSPGQSITISCTGTSSDVGGYNYVSWYQQHPGKAPKLMIYDVTTRPSGV
SDRFSGSKSGNTASLTISGLQAEDEADYYCSSYSSGSTPALFGGGTQLTVL
(SEQ ID NO:16);

TRL1230 heavy chain variable region has the amino acid sequence:

QVQLVQSGGGLVKPGGSLRLSCGASGFNLSSYSMNWVRQAPGKGLEWVSSISSRSSYIY
YADSVQGRFTISRDNAKNSLYLQMNSLRAEDTAIYYCARVSPSTYYYYGMDVWGQGT
TVTVSS (SEQ ID NO:17);

TRL1230 light chain lambda variable region has the amino acid sequence:

DIVLTQPSSVSVSPGQTARITCSGDELPKQYAYWYQQKPGQAPVLVIYKDNERPSGIPER
FSGSSSGTTVTLTISGVQAEDEADYYCQSADSSGTYVVFGGGTKLTVL (SEQ ID NO:18);

TRL1232 heavy chain variable region has the amino acid sequence:

QVQLVESGAEVKKPGALVKVSCKASGYTFSGYYMHWVRQAPGQGLEWMGWINPKSG
GTKYAQKFQGRVTMTRDTSISTAYMELSRLRSDDTAVYFCARGGPSNLERFLERLQPRY
SYDDKYAMDVWGQGTTVTVSS (SEQ ID NO:19);

TRL1232 light chain kappa variable region has the amino acid sequence:

DIVMTQSPGTLSLSPGARATLSCRASQSVSSIYLAWYQQKPGQAPRLLIFGASSRATGIPD
RFSGSGSGTDFTLTISRLEPEDFAVYYCQQYGSSPYTFGQGTKLEIKR (SEQ ID NO:20);

TRL1242 heavy chain variable region has the amino acid sequence:

QVQLVQSGTEVKKPGESLKISCEGSRYNFARYWIGWVRQMPGKGLDWMGIIYPGDSDT
RYSPSFQGQVSISADKSISTAYLQWNSLKASDTAMYYCARLGSELGVVSDYYFDSWGQ
GTLVTVSS (SEQ ID NO:21);

TRL1242 light chain kappa variable region has the amino acid sequence:

DIVLTQSPDSLAVSLGERATINCKSSQSVLDRSNNKNCVAWYQQKPGQPPKLLIYRAAT
RESGVPDRFSGSGSGTDFSLTISSLQAEDVAVYFCQQYYSIPNTFGQGTKLEIKR
(SEQ ID NO:22); and

TRL1245 heavy chain variable region has the amino acid sequence:

QVQLVESGGGLVKAGGSLRLSCVASGFTFSDYYMSWIRQAPGKGLEWISFISSSGDTIFY
ADSVKGRFTVSRDSAKNSLYLQMNSLKVEDTAVYYCARKGVSDEELLRFWGQGTLVT
VSS (SEQ ID NO:23);

TRL1245 light chain variable region has the amino acid sequence:

DIVLTQDPSVSVSPGQTARITCSGDALPKKYAYWYQQKSGQAPVLVIYEDTKRPSGIPER
FSGSSSGTVATLTISGAQVEDEADYYCYSTDSSGNQRVFGGGTKLTVL (SEQ ID NO:24).

The encoding nucleotide sequences for the variable regions of TRL295 are:

Heavy Chain: caggtgcagctggtgcagtctgggggaggcttggtacagcctggggggtccctgagactctcctgtgcag
cctctggatttaccttcagtgattatagtatgaactgggtccgccaggctccagggaagggactggaatggctttcatacattagtcacactatt
actaccatatactacgccgactctgtgaagggccgattcaccatctccagagacaatgccgacagctcactgtatctccaaatgaacagcct
gggagacgaggacacggctgtgtattactgtgcgagagctccattagtaaactgtagtactagtggctgccagtccggaagctggttcgac
acctggggccagggaaccctggtcaccgtctcctca (SEQ ID NO:25);

Light Chain: gatatcgagctgactcaggcaccctcggtgtcagtgtatccaggacagacggccaggatcacctgctctgga
gatgcactgccaaagcaatatgcttattggtaccagcagaagccaggccaggcccctgtggtggtgatatataaagacagtgagaggccct
cagggatctctgagcgattctctggctccagctcggggacaacagtcacgttgaccatcagtggagtccaggcaggggacgaggctgact
attattgtcaatcagttgacaccagtgtttcttattatgtggtcttcggcggagggaccaagttgaccgtccta (SEQ ID NO:26).

The encoding nucleotide sequences for the variable regions of TRL1012 are:

Heavy Chain: caggtgcagctggtggagtccgggggaggcttggtacagcctggggggtccctgagactttcctgtgccg
cctctggattccccttcagtagttatgccatgagttgggtccgtcaggctccagggaaggggctggagtgggtctcagccatcagtggcaac
ggcgctgactcatattacgcagactccgtgaagggccgcttcaccacttccagagacaagtccaagaatacagtttatttgcaaatgaacag
actcagggccgaggacacggccgtatattactgtgcgaaagatatgcgacggtatcattatgacagtagtggtctgcacttctggggccagg
gaaccctggtcaccgtctcctca (SEQ ID NO:27);

Light Chain: gatatcatgctgactcagccccctcagtgtctgcggcccccggacagaaggtcaccatctcctgctctggaa
gcagctccaacattgggacgaattatgtgtcctggttccagcaggtcccaggaacagcccccaaattcctcatttatgacaattataaacgac
cctcagaaactcctgaccgattctctggctccaagtctggcacgtcggccaccctggacatcaccggactccagactggggacgaggcca
attattactgcgcaacatgggacagtagcctgagtgcttgggtgttcggcggagggaccaaggtgaccgtcctg
(SEQ ID NO:28).

The encoding nucleotide sequences for the variable regions of TRL1068 are:

Heavy Chain: caggtgcagctggtggagtccggcccaggactggtgaagccttcggagaccctgtccctcacctgcaggg
tctctggtgactccaatcggccttcctactggagctggatcaggcaggccccagggaaggcaatggagtggataggttatgtctatgacagt
ggggtcaccatctacaatccctccctcaagggtcgagtcacaatatcactagacacgtcgaagacgcggttctccctgaaactgacctctgt
gatcgctgcggacacggccgtatattattgtgcgcgagaacgttttgatcggacatcgtataagagttggtggggccagggaacgcaggtc
accgtctcctca (SEQ ID NO:29);

Light Chain: gatatcgtgctgactcaggccccaggcactctgtctttgtctccaggggacagagccaccctctcctgtaggg
ccagtcagcgtcttggcggcacgtccttagcctggtaccagcacagatctggccaggctcccaggctcatcctctacggaacttcaaacag
ggccactgacacccctgacaggtttagtggcagtgggtctgggacagacttcgttctcaccatcagttccctggagcctgaagattttgcagt
gtattactgtcagcaatatggcagcccaccgtacacttttggccaggggaccactctggacatcaaa (SEQ ID NO:30).

The encoding nucleotide sequences for the variable regions of TRL1070 are:

Heavy Chain: caggtgcagctggtgcagtctgggggaaccttggtccagccgggggggtccctgagactctcctgtgcag
cctctggattcacctttagttactactcgatgagctgggtccgccaggctccagggaaggggctggagtgggtggccaacataaagcacga
tggaactgagagaaattatgtggactctgtgaagggccgattcaccatctccagagacaacagcgagaagtctctttacctgcaaatgaaca
gcctgagagccgaggacacggctgtgtattactgtgcgaagtattattatggtgccgggactaattatccccttaagtactggggccaggga
acccgggtcaccgtctcctca (SEQ ID NO:31);

Light Chain: gatatcctgatgacccagtctccatcctccctgtctgcatctgtaggagacagagtcaccatcacttgccgggc
aagtcagggcattagaaatgatttaggctggtatcagcagaaaccagggaaagcccctaagctcctgatctatgctgcatccagtttacaaa
gtggggtcccatcaaggttcagcggcagtggatctggcacagatttcactctcaccatcagcagcctgcagcctgaagattttgcaacttatt
actgtctacaagattacaattacccgctcactttcggcggagggaccaaggtggagatcaaacga (SEQ ID NO:32).

The encoding nucleotide sequences for the variable regions of TRL1087 are:

Heavy Chain: caggtgcagctgctcgagtcaggcccaggcctggttaggccctcggacaccctgtccctcacctgcactttt
tccgctgacctcagcaccaacgcctattggacctggatccggcagcccccaggaaagggactggagtggattggctatatgtctcatagtg
gggaagggattacaatccctccttcaaccggcgagtcaccatttcagtggacacgtcgaagaaccaggttttcttgaggctgacgtcagtg
acctctgcggacacggccgtctatttctgtgtgagagaagtcggcagttactacgactactggggccagggaatcctggtcaccgtctcctc
a (SEQ ID NO:33);

Light Chain: gatatcgagatgacccagtctccatcctctttgtctgcatctgtcggagacagaatcaccatcacttgtcgggcg
agtcagggtattagcacctggttagcctggtatcagcagaaaccggggaaagcccctaagtccctgatcttttctacgtccagcctgcatagt
ggggtcccctcaaagttcagcggcagtgggtctgggacagacttcactctcaccatcaccaacctgcagcctgaagattttgcaacttattac
tgccaacagaaatgggagaccccttatagttttggccaggggaccaagctggacatgatacga (SEQ ID NO:34).

The encoding nucleotide sequences for the variable regions of TRL1215 are:

Heavy Chain: caggtgcagctggtggagtctggaactgaggtgaagaaccctggagcctcagtgaaggtctcctgcacgg
cctctggttacaaatttgacgaatatggtgtcagttgggtgcgacagtcccctggacaaggacttgagtggatgggatggatcagtgtttataa
tggcaagacaaactatagccagaactttcagggcagactcaccctgaccacagagacatccaccgacacagcctacatggagcttacgag
cctcagacctgacgacacggccgtctattactgtgcgacagacaaaaactggttcgacccctggggcccgggaaccctggtcaccgtctc
ctca (SEQ ID NO:35);

Light Chain: gatatcgtgatgacccagtctccctccgcgtccgggtctcctggacagtcaatcaccatctcctgcactggaac
caacactgattataattatgtttcctggtaccagcaccaccccggcaaagcccccaaagtcattatttatgacgtcaaaaagcggccctcggg
ggtccctagtcgcttctctggctccaggtctggcaacacggccaccctgaccgtctctgggctccagactgaggatgaggctgattattattg
tgtctcatatgcagacaacaatcattatgtcttcggaagtgggaccaaggtcaccgtcctg (SEQ ID NO:36).

The encoding nucleotide sequences for the variable regions of TRL1216 are:

Heavy Chain: caggtgcagctggtggagtccgggggaggcgtggtccagcctggagggtccctgagagtctcctgtgca
gcctctgcgttcagtttcagggattatggcatacactgggtccgccaggctccaggcaaggggctgcaatgggtggcggttatttcacatgat
ggaggtaagaaattctatgcagactccgtgaggggccgattcaccatctccagagacaattccgagaacacactgtatctccaaatgaaca
gcctgagatctgacgacacggctgtctattactgtgcgaggctcgttgccagttgcagtggttccacctgcacaacgcaacctgctgcctttg
acatttggggcccagggacattggtcaccgtctcttca (SEQ ID NO:37);

Light Chain: gatatcatgctgactcagccgccctcggtgtcagtgtccccaggacaaacggccaggatcacctgctctgga
gatgcattgccaaaaaaatatacttattggtatcagcagaagtcaggccaggcccctgttctgctcatctatgaggacaggaaacgaccctcc
gagatccctgagagattctctgccttcacctcatggacgacggccaccttgactatcactgggggcccaggtgagagatgaagctgactacta
ctgttattcaacagacatcagtggtgatataggagtgttcggcggagggaccaagctgaccgtccta (SEQ ID NO:38).

The encoding nucleotide sequences for the variable regions of TRL1218 are:

Heavy Chain: gatatcgtgctgactcagtcggcctccgtgtctgggtctcctggacagtcgatcaccatctcctgcactggaa
ccagcagtgacgttggtggatataactatgtctcctggtaccaacaacacccaggcaaagcccccaaactcatgatttatgatgtcactactc
ggccttcaggggtttctgatcgcttctctggctccaagtctggcaacacggcctccctgaccatctctgggctgcaggctgaggacgaggct
gattattattgcagctcatattcaagcggctccacacctgctctgtttgggggggcacccagctgaccgtcctc (SEQ ID NO:39);

Light Chain: gatatcgtgctgactcagtcggcctccgtgtctgggtctcctggacagtcgatcaccatctcctgcactggaac
cagcagtgacgttggtggatataactatgtctcctggtaccaacaacacccaggcaaagcccccaaactcatgatttatgatgtcactactcg
gccttcaggggtttctgatcgcttctctggctccaagtctggcaacacggcctccctgaccatctctgggctgcaggctgaggacgaggctg
attattattgcagctcatattcaagcggctccacacctgctctgtttgggggggcacccagctgaccgtcctc (SEQ ID NO:40).

The encoding nucleotide sequences for the variable regions of TRL1230 are:

Heavy Chain: caggtgcagctggtgcagtctgggggaggcctggtcaagcctggggggtccctgagactctcctgtgcag
cctctggatttaacctcagtagttatagcatgaactgggtccgccaggctccagggaaggggctggagtgggtctcatccattagtagtaga
agtagttacatatactatgcagactcagtgcagggccgattcaccatctccagagacaacgccaagaactcactgtatctgcaaatgaacag
cctgagagccgaggacacggctatatattactgtgcgagagtatctccgtccacctattattattatggtatggacgtctggggccaagggac
cacggtcaccgtctcctca (SEQ ID NO:41);

Light Chain: gatatcgtactcactcagccgtcctcggtgtcagtgtccccaggacagacggccaggatcacctgctctgga
gatgaattgccaaagcaatatgcttattggtaccagcagaagccaggccaggcccctgtgttggtaatatataaagacaatgagaggccctc
agggatccctgagcgattctctggctccagctcagggacaacagtcacgttgaccatcagtggagtccaggcagaagacgaggctgacta
ttactgtcaatcagcagacagtagtggtacttatgtggtgttcggcggagggaccaagctgaccgtccta (SEQ ID NO:42).

The encoding nucleotide sequences for the variable regions of TRL1232 are:

Heavy Chain: caggtgcagctggtggagtctggggctgaggtgaagaagcctggggccttagtgaaggtctcctgcaagg
cttctggatacaccttcagcggctactatatgcactgggtgcgacaggcccctggacaagggcttgagtggatgggatggatcaaccctaa
gagtggtggcacaaagtatgcacagaagtttcagggccgggtcaccatgaccagggacacgtccatcagcacagcctacatggagttga
gcaggctaagatctgacgacacggccgtgtatttctgtgcgagaggcggaccttcaaatttggaacgatttttggagaggttacaaccccgct
acagttacgacgacaagtatgctatggacgtctggggccaagggaccacggtcaccgtctcctca (SEQ ID NO:43);

Light Chain: gatatcgtgatgacccagtctccaggcaccctgtctttgtctccaggggcaagagccaccctctcctgcaggg
ccagtcagagtgttagcagcatctatttagcctggtaccagcagaaacctggccaggctcccaggctcctcatctttggtgcatccagcagg
gccactggcatcccagacaggttcagtggcagtgggtctgggacagacttcactctcaccatcagcagactggagcctgaagattttgcagt
gtattactgtcagcagtatggtagctcaccgtacacttttggccaggggaccaagctggagatcaaacgaa (SEQ ID NO:44).

The encoding nucleotide sequences for the variable regions of TRL1242 are:

Heavy Chain: caggtgcagctggtgcagtctggaacagaagtgaaaaagcccggggagtctctgaagatctcctgtgagg
gttctcgatacaactttgccaggtactggatcggctgggtgcgccagatgcccggaaaaggcctggactggatggggatcatctatcctggt

gactccgataccagatacagcccgtccttccaaggccaggtcagcatctcagccgacaagtccatcagtaccgcctacctgcagtggaaca
gcctgaaggcctcggacaccgccatgtattattgtgcgagacttgggagcgagcttggagtggtctctgattattactttgactcctggggcc
agggaaccctggtcaccgtctcctca (SEQ ID NO:45);

Light Chain: gatatcgtgttgactcagtctccagactccctggctgtgtctctgggcgagagggccaccatcaactgcaagtc
cagccagagtgttttagacaggtccaacaataagaactgtgtagcttggtaccagcagaaaccgggacagcctcctaaactgctcatttacc
gggctgctacccgggaatccggggtccctgatcgattcagtggcagcgggtctgggacagacttcagtctcaccatcagcagcctgcagg
ctgaagatgtggcagtttatttctgtcagcaatattatagtattccgaacacttttggccaggggaccaagctggagatcaaacga
(SEQ ID NO:46).

The encoding nucleotide sequences for the variable regions of TRL1245 are:

Heavy Chain: caggtgcagctggtggagtctgggggaggcttggtcaaggctggagggtccctgagactctcctgtgtagc
ctctggattcaccttcagcgactactacatgtcctggattcgccaggctccagggaaggggctggagtggatttcatttattagtagtagtggt
gataccatattttacgcagactctgtgaagggccgattcaccgtctccagggacagcgccaagaactcactgtatcttcaaatgaacagcctg
aaagtcgaggacacggccgtgtattactgtgcgaggaagggggtgtccgacgaggaactactgcgcttctggggccagggaaccctggt
caccgtctcctca (SEQ ID NO:47);

Light Chain: gatatcgtgctgactcaggacccctcggtgtcagtgtccccaggacaaacggccaggatcacctgctctgga
gatgcattgccaaaaaaatatgcttattggtaccagcagaagtcaggccaggcccctgtgctggtcatctatgaggacaccaaacgaccctc
cgggatccctgagagattctctggctccagctcagggacagtggccaccttgactatcagtggggcccaggtggaggatgaagctgacta
ctattgttactcaacagacagcagcggtaatcagagggtattcggcggagggaccaagctgaccgtccta (SEQ ID NO:48).

### Example 2

### Determination of Affinity

For practice of the assay method, ∼1 mg of IHF was required. IHF is difficult to express in bacteria (since it has a dual function involving gene regulation, leading to toxicity to bacteria expressing high levels). Obtaining sufficient material for mAb discovery from bacterial sources is thus difficult (and expensive). The protein was therefore expressed in HEK293 (mammalian) cells, with a poly-histidine tag to enable easy purification. The homologs from *Staphylococcus aureus* (Sa), *Pseudomonas aeruginosa* (Pa), *Klebsiella pneumoniae* (KP) and *Haemophilus influenzae* (Hi) were all prepared in this manner. These four are of particular utility since they span a substantial portion of the diversity in sequences of the DNABII family.

TRL295 was shown to bind with high affinity to the IHF peptide of *H. influenzae* and moreover to bind to IHF from additional bacterial species.

The chart below shows the degree of identity to *Haemophilus* of various IHF and HU proteins from a variety of bacterial species.

| Species | Protein | Sequence Identity to *Haemophilus* |
|---|---|---|
| *Haemophilus influenzae* | IHF alpha | 100 |
| *Escherichia coli* | IHF alpha | 67 |
| *Enterobacter cloacae* | IHF alpha | 66 |
| *Enterobacter aerogenes* | IHF alpha | 66 |
| *Klebsiella oxytoca* | IHF alpha | 65 |
| *Pseudomonas aeruginosa* | IHF alpha | 61 |
| *Acinetobacter baumannii* | IHF alpha | 58 |
| *Streptococcus pneumoniae* | HU | 38 |
| *Staphylococcus aureus* | HU | 38 |

Further, the high affinity binding of TRL295 was shown to be retained even as the pH was decreased from physiological (pH 7.5) to pH 4.5, as shown below.

| pH | Kd (nM) |
|---|---|
| 7.5 | 4.2 |
| 6.5 | 2.8 |
| 5.5 | 2.8 |
| 4.5 | 3.7 |
| 3.5 | no binding |
| 2.5 | no binding |

This is important since bacteria often secrete lactic acid which reduces the local micro-environment pH as a way of inhibiting immune system attack.

The chart below shows the results of ELISA assays to determine binding of various DNABII proteins. The numbers represent OD values which are useful for comparison to TRL1068 - higher values represent higher binding affinity. TRL1068 shows similar binding to all four homologs, but low binding to BSA, as does TRL1215. The abbreviations are

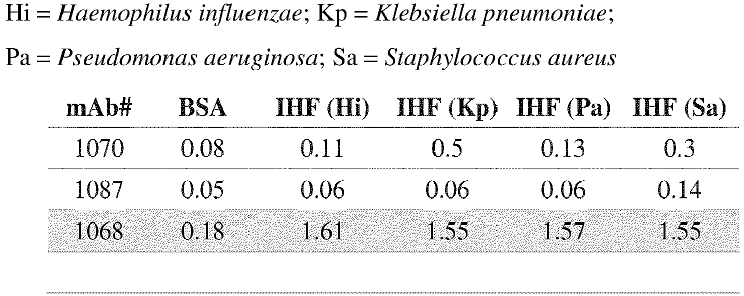

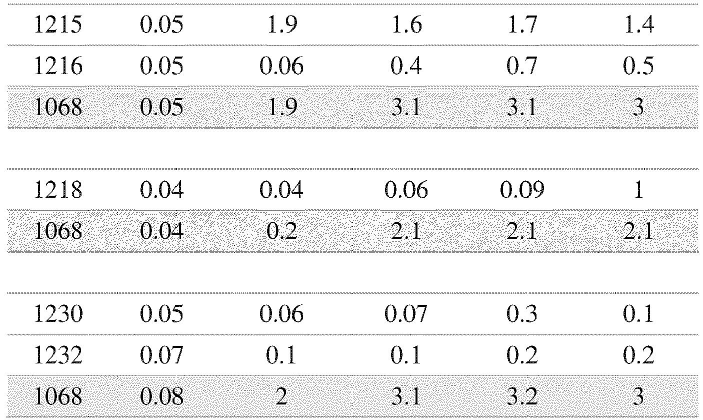

The affinity of TRL1068 for the target protein was directly determined using a ForteBio Octet™ biosensor model QK (Pall Corporation; Menlo Park, CA) with Kd determined by standard methods for measuring ratio of on and off rates (Ho D, et al., BioPharm International (2013) 48-51). The values were: 1 nM for *Staphylococcus aureus* (Sa), 1 nM for *Pseudomonas aeruginosa* (Pa), 7 nM for *Klebsiella pneumoniae* (Kp) and 350 nM for *Haemophilus influenzae* (Hi).

### Example 3

### Epitope Selection for Focused mAb Discovery

Computational methods for analyzing the likelihood of antigenicity (induction of antibody responses) are known in the art (reviewed by J. Ponomarenko, et al., in BMC Bioinformatics (2008) 9:514). Using an improved variation of these published methods, a map of the likely epitopes was generated for the IHF from *Haemophilus influenzae* from a model of the structure based on the published structure found in the Protein Data Bank (pdb 1OWF) (Figure 1B). For the display in Figure 1A, a value was assigned to the residue at the midpoint of each 11-amino acid segment. A value above 0.9 denotes a region with high likelihood of being susceptible to antibody binding.

Three regions were identified as having high likelihood of being recognized by antibodies: residues 10-25, 56-78, and 86-96.

As illustrated in Figure 2, the region from residues 56-78 of the IHF protein is substantially conserved across multiple clinically important bacterial species. Structural modeling of IHF from multiple species has confirmed that the homology is high, particularly in the DNA binding region (Swinger, K. K., et al., Current Opinion in Structural Biology (2004) 14:28-35). Peptides that only partially overlap with this optimal region are less likely to fold spontaneously into the relevant three dimensional conformation and will be more difficult to chemically crosslink in order to lock in that conformation. Optimizing the fidelity to the native protein in this manner is advantageous for both mAb discovery and for use of the peptide as an immunogen.

As noted above, the epitopes thus identified are positions 10-25 *of H. influenzae* IHF: IEYLSDKYHLSKQDTK (SEQ ID NO:49); positions 56-78 *of H. influenzae* IHF: RDKSSRPGRNPKTGDVVAASARR (SEQ ID NO:50); and positions 86-96 *of H. influenzae* IHF: QKLRARVEKTK (SEQ ID NO:51).

Figure 3A shows a computational construction of the IHF dimer complexed with DNA. The B cell epitopes of the invention are shown in Figure 3B. Figure 3A shows that the epitopes are partially masked by DNA when bound. However, if exposed, these portions of the proteins may generate antibodies of high affinity capable of binding them and thus preventing the formation of biofilm or causing an established biofilm to lose structural integrity as the DNABII protein is sequestered by the antibody. Other sites on the DNABII protein may also suffice to achieve extraction of the protein out of the biofilm based on higher affinity binding by the mAb as compared to the protein's affinity for components of the biofilm.

### Example 4

### In Vitro Bioactivity Assessment

TRL1068 was tested for bioactivity using a commercial assay from Innovotech (Edmonton, Alberta; Canada). Biofilms were formed in multiple replicates on pins in a 96-well microplate format exposed to media including *Pseudomonas aeruginosa* (ATCC 27853) or *Staphylococcus aureus* (ATCC 29213). Following biofilm formation, the pins were treated in different wells with a non-immune isotype control mAb or with TRL1068 at 1.2 µg/mL (-10 nM) for 12 hours. As evident in the scanning electron micrographs of the treated surfaces in Figure 4, TRL1068 was highly effective at dissolving the biofilm. These results establish that the mAb can degrade the biofilm, thereby removing the attached bacteria.

### Example 5

### In Vivo Bioactivity Assessments

Several animal models exist for evaluation of activity. For example, at University Hospital Basel (Switzerland), a model for biofilm on implanted prostheses involves implanting Teflon® tissue cages (Angst+Pfister; Zurich, Switzerland) subcutaneously in BALB/c mice, which are then allowed to heal for 2 weeks. After confirming sterility of the cage by extracting fluid from it, the site is infected with 4 x 10³ CFU (colony-forming units) of *S*. *aureus* (ATCC 35556), an inoculum mimicking a perioperative infection. After 24 hours, the site is injected with drug. After 72 hours, the mice are sacrificed and the tissue cage recovered. Viable bacteria are counted by plating on blood agar (Nowakowska J., et al., Antimicrob Agents Chemother (2013) 57:333).

A second example is a model that involves inducing biofilm on heart valves, mimicking native valve endocarditis (Tattevin P., et al., Antimicrob Agents Chemother (2013) 57:1157). New Zealand white rabbits are anesthetized. The right carotid artery is cut and a polyethylene catheter is positioned across the aortic valve and secured in place. Twenty four hours later, 1 mL of saline plus 8 x 10⁷ CFU of S. *aureus* is injected through the catheter, which induces a biofilm infection in 95% of the animals. Drugs (anti-biofilm and antibiotic) are administered i.v. and efficacy is evaluated after 4 days by tissue pathology and blood bacterial levels.

### Embodiments of the invention:

1. A monoclonal binding moiety that has affinity for at least one DNABII protein that exceeds the affinity of said protein for components of a biofilm that includes said DNABII protein, which is a monoclonal antibody (mAb), an aptamer, a non-Ig scaffold or a structured short peptide.
2. The binding moiety of embodiment 1 wherein the mAb is an antigen binding fragment, an Fv antibody, or a complete antibody.
3. The binding moiety of embodiment 1 wherein the biofilm component is branched DNA, or wherein the DNABII protein is IHF or a subunit thereof, or is HU protein or is DPS or is Hfq or is CbpA or CbpB.
4. The binding moiety of embodiment 1 or 2 which is an mAb which is a human or humanized mAb or a feline, canine, equine, bovine, caprine or ovine mAb.
5. The binding moiety of embodiment 1 or 2 which is an mAb wherein the variable region comprises
   (a) the CDR regions of the heavy chain of TRL295 (SEQ ID NO:1); or
   (b) the CDR regions of the heavy chain of TRL1012 (SEQ ID NO:3); or
   (c) the CDR regions of the heavy chain of TRL1068 (SEQ ID NO:5); or
   (d) the CDR regions of the heavy chain of TRL1070 (SEQ ID NO:7); or
   (e) the CDR regions of the heavy chain of TRL1087 (SEQ ID NO:9); or
   (f) the CDR regions of the heavy chain of TRL1215 (SEQ ID NO:11); or
   (g) the CDR regions of the heavy chain of TRL1216 (SEQ ID NO:13); or
   (h) the CDR regions of the heavy chain of TRL1218 (SEQ ID NO:15); or
   (i) the CDR regions of the heavy chain of TRL1230 (SEQ ID NO:17); or
   (j) the CDR regions of the heavy chain of TRL1232 (SEQ ID NO:19); or
   (k) the CDR regions of the heavy chain of TRL1242 (SEQ ID NO:21); or
   (l) the CDR regions of the heavy chain of TRL1245 (SEQ ID NO:23).
6. The mAb of embodiment 5 wherein
   the mAb of (a) further comprises the CDR regions of the light chain of TRL295 (SEQ ID NO:2) ;or
   the mAb of (b) further comprises the CDR regions of the light chain of TRL1012 (SEQ ID NO:4); or
   the mAb of (c) further comprises the CDR regions of the light chain of TRL1068 (SEQ ID NO:6); or
   the mAb of (d) further comprises the CDR regions of the light chain of TRL1070 (SEQ ID NO:8); or
   the mAb of (e) further comprises the CDR regions of the light chain of TRL1087 (SEQ ID NO:10); or
   the mAb of (f) further comprises the CDR regions of the light chain of TRL1215 (SEQ ID NO:12); or
   the mAb of (g) further comprises the CDR regions of the light chain of TRL1216 (SEQ ID NO:14); or
   the mAb of (h) further comprises the CDR regions of the light chain of TRL1218 (SEQ ID NO:16); or
   the mAb of (i) further comprises the CDR regions of the light chain of TRL1230 (SEQ ID NO:18); or
   the mAb of (j) further comprises the CDR regions of the light chain of TRL1232 (SEQ ID NO:20); or
   the mAb of (k) further comprises the CDR regions of the light chain of TRL1242 (SEQ ID NO:22); or
   the mAb of (1) further comprises the CDR regions of the light chain of TRL1245 (SEQ ID NO:24).
7. The mAb of embodiment 1 which comprises
   (a) the variable region of the heavy chain of TRL295 (SEQ ID NO:1); or
   (b) the variable region of the heavy chain of TRL1012 (SEQ ID NO:3); or
   (c) the variable region of the heavy chain of TRL1068 (SEQ ID NO:5); or
   (d) the variable region of the heavy chain of TRL1070 (SEQ ID NO:7); or
   (e) the variable region of the heavy chain of TRL1087 (SEQ ID NO:9); or
   (f) the variable region of the heavy chain of TRL1215 (SEQ ID NO:11); or
   (g) the variable region of the heavy chain of TRL1216 (SEQ ID NO:13); or
   (h) the variable region of the heavy chain of TRL1218 (SEQ ID NO:15); or
   (i) the variable region of the heavy chain of TRL1230 (SEQ ID NO:17); or
   (j) the variable region of the heavy chain of TRL1232 (SEQ ID NO:19); or
   (k) the variable region of the heavy chain of TRL1242 (SEQ ID NO:21); or
   (l) the variable region of the heavy chain of TRL1245 (SEQ ID NO:23).
8. The mAb of embodiment 7 wherein
   the mAb of (a) further comprises the variable region of the light chain of TRL295 (SEQ ID NO:2); or
   the mAb of (b) further comprises the variable region of the light chain of TRL1012 (SEQ ID NO:4); or
   the mAb of (c) further comprises the variable region of the light chain of TRL1068 (SEQ ID NO:6); or
   the mAb of (d) further comprises the variable region of the light chain of TRL1070 (SEQ ID NO:8); or
   the mAb of (e) further comprises the variable region of the light chain of TRL1087 (SEQ ID NO:10); or
   the mAb of (f) further comprises the variable region of the light chain of TRL1215 (SEQ ID NO:12); or
   the mAb of (g) further comprises the variable region of the light chain of TRL1216 (SEQ ID NO:14); or
   the mAb of (h) further comprises the variable region of the light chain of TRL1218 (SEQ ID NO:16); or
   the mAb of (i) further comprises the variable region of the light chain of TRL1230 (SEQ ID NO:18); or
   the mAb of (j) further comprises the variable region of the light chain of TRL1232 (SEQ ID NO:20); or
   the mAb of (k) further comprises the variable region of the light chain of TRL1242 (SEQ ID NO:22); or
   the mAb of (1) further comprises the variable region of the light chain of TRL1245 (SEQ ID NO:24).
9. A method to prevent formation of or to dissolve a biofilm associated with an industrial process which method comprises treating a surface susceptible to or containing a biofilm with the binding moiety of any one of embodiments 1-8.
10. The binding moiety of any one of embodiments 1-8 or, if the binding moiety is a protein a nucleic acid encoding said protein, for use in a method to dissolve biofilm topically on a subject or to confer passively on a subject a capability to dissolve biofilms.
11. A recombinant expression system for producing a binding moiety of any one of embodiments 1-8 wherein said binding moiety is a protein, wherein said expression system comprises a nucleotide sequence encoding said protein operably linked to control sequences for expression.
12. Recombinant host cells that have been modified to contain the expression system of embodiment 11.
13. A method to prepare a protein-binding moiety that binds a DNABII protein which method comprises culturing the cells of claim 12.
14. A method to identify a binding moiety that has affinity with respect to at least one DNABII protein greater than the affinity of a biofilm component for said DNABII protein which method comprises contacting a candidate binding moiety with a biofilm component and with said at least one DNABII protein, and determining the ratio of said DNABII protein bound to said binding moiety as compared to DNABII bound with the biofilm component, whereby a ratio greater than one identifies a binding moiety that has affinity with respect to at least one DNABII protein greater than the affinity of a biofilm component for said DNABII protein.
15. A method to identify a binding moiety with utility in acidic environments which method comprises measuring the affinity of a candidate for a DNABII protein over a range of pH conditions whereby a candidate moiety with low nanomolar affinity at pH 4.5 is identified as having utility in acidic environments.
16. A method to identify an agent that reverses drug resistance in multiple species of bacteria which method comprises evaluating an agent for activity in disrupting biofilms produced by multiple species, wherein an agent which disrupts said biofilms is identified as an agent that reverses drug resistance.
17. A method to identify an agent that reverses drug resistance in multiple species of bacteria which method comprises evaluating an agent for binding to DNABII proteins characteristic of a multiplicity of microbial species wherein an agent that binds a multiplicity of said proteins is identified as an agent that reverses drug resistance.
18. The method of embodiment 17 wherein said DNABII proteins are produced in mammalian cells.
19. A peptide that consists of the amino acid sequence IEYLSDKYHLSKQDTK (SEQ ID NO:49) (positions 10-25 of the *Haemophilus influenzae* IHFα chain) or the amino acid sequence DKSSRPGRNPKTGDVVAASARR (SEQ ID NO:50) (positions 56-78 of the *Haemophilus influenzae* IHFα chain) or the amino acid sequence KLRARVEKTK (SEQ ID NO:51) (positions 86-96 of the *Haemophilus influenzae* IHFα chain), or homologs thereof.
20. The peptide of embodiment 17 which further contains heterologous amino acid sequence or is coupled to a heterologous non-peptide moiety.
21. A method to obtain antibodies immunoreactive with IHF protein or to generate B cells that secrete antibodies immunoreactive with IHF protein which method comprises administering the peptide of embodiment 19 or 20 to a subject; and recovering antibodies from said subject; or recovering B cells from said subject.
22. The method of embodiment 21 which further comprises screening said B cells for secretion of an antibody with high affinity for IHF protein thus identifying B cells that secrete antibodies immunoreactive with IHF; and isolating DNA or mRNA encoding said antibodies from said cells.
23. A method to treat a condition in a subject characterized by the formation of a biofilm in said subject which method comprises treating said subject with the binding moiety of any one of embodiments 1-8.
24. The method of embodiment 23 wherein said condition is heart valve endocarditis, chronic non-healing wounds, including venous ulcers and diabetic foot ulcers, ear infections, sinus infections, urinary tract infections, pulmonary infections, cystic fibrosis, chronic obstructive pulmonary disease, catheter-associated infections, infections associated with implanted prostheses, and periodontal disease.
25. A method to treat a condition in a subject characterized by formation of a biofilm in said subject which method comprises administering to said subject a composition comprising the peptide of either embodiment 19 or 20 or a nucleic acid encoding said peptide.
26. The method of embodiment 25 wherein said condition is heart valve endocarditis, chronic non-healing wounds, including venous ulcers and diabetic foot ulcers, ear infections, sinus infections, urinary tract infections, pulmonary infections, cystic fibrosis, chronic obstructive pulmonary disease, catheter-associated infections, implanted prostheses infections, and periodontal disease.
27. A method to produce a DNABII protein which method comprises culturing mammalian cells that have been modified to contain an expression system for said DNABII protein.
28. A method to identify an immunogen useful to elicit the formation of antibodies for treating biofilm, which method comprises screening a library of candidate immunogens for high affinity binding to a binding moiety of any of embodiments 1-8, whereby a candidate member of the library that binds with high affinity to said binding moiety is identified as said immunogen.
29. A method to obtain a binding moiety that has an affinity for at least one DNABII protein that exceeds the affinity of said protein for components of a biofilm that includes said DNABII protein which method comprises administering to a subject an immunogen identified by the method of embodiment 28.

## Claims

1. A monoclonal antibody (mAb) which has affinity for residues 56-78 of an IHF protein, which mAb includes an antigen binding fragment, an Fv antibody, a bispecific antibody, and a complete antibody.

2. The mAb of claim 1 wherein said mAb is a human mAb or a feline, canine, equine, bovine, caprine or ovine mAb.

3. A method to prevent formation of, or to disrupt *in situ* a biofilm associated with an industrial process, which method comprises treating a surface susceptible to the biofilm or a surface containing the biofilm with the mAb of claim 1 or 2.

4. Recombinant host cells that have been modified to contain a recombinant expression system for producing the mAb of claim 1 or 2, wherein said expression system comprises a nucleotide sequence encoding said mAb operably linked to control sequences for expression.

5. A method to prepare an mAb that binds the IHF protein, which method comprises culturing the cells of claim 4 and recovering said mAb.

6. The mAb of any of claim 1 or 2 for use in a method to treat a condition in a subject **characterized by** the formation of a biofilm in said subject.

7. The mAb of claim 6, wherein said condition is heart valve endocarditis, chronic non-healing wounds including venous ulcers and diabetic foot ulcers, ear infections, sinus infections, urinary tract infections, pulmonary infections, cystic fibrosis, chronic obstructive pulmonary disease, catheter-associated infections, infections associated with implanted prostheses, and periodontal disease.
